# EUROPÄISCHE PATENTANMELDUNG

(11) **EP 3 510 964 A1**
(43) Veröffentlichungstag der Anmeldung: **17.07.2019**
(21) Anmeldenummer: 18210656.7
(22) Anmeldetag: 14.11.2016
(51) Int. Cl.: A61B 90/70, A61B 34/30, A61B 17/00

(54) **BEWEGUNGSVORRICHTUNG SOWIE SYSTEM ZUR REINIGUNG VON MEDIZINISCHEN INSTRUMENTEN**

(30) Priorität: 13.11.2015 DE 202015106167 U
(62) Teilanmeldung aus: 16198744.1
(71) Anmelder: BANDELIN patent GmbH & Co. KG, 12207 Berlin (DE)
(72) Erfinder: JUNG, Rainer, 13125 Berlin (DE); HELKE, Juliane, 12527 Berlin (DE); MÖHRICKE, Jonas, 10319 Berlin (DE); HÄSEN, Claudia, 16727 Oberkrämer (DE); BIERMANN, Ferdinand, 52066 Aachen (DE); HOPPENAU, Sören, 12103 Berlin (DE)
(74) Vertreter: Pfenning, Meinig & Partner mbB

(57) **Zusammenfassung**

Das vorliegende Schutzrecht befasst sich mit Systemen zur Reinigung von medizinischen Geräten, beispielsweise enthaltend eine Bewegungsvorrichtung mit einem Adapter (2) zur Ankopplung mindestens eines medizinischen Instruments (3), wobei der Adapter (2) mindestens ein mittels eines Antriebs bewegbares Kopplungselement (4) umfasst. Das Kopplungselement (4) ist so gestaltet, dass bei Ankopplung des medizinischen Instruments (3) zumindest ein Bereich des medizinischen Instruments (3) durch Bewegung des Kopplungselements (4) in Bewegung versetzbar ist. Außerdem enthält das System eine Schwenk- und/oder Hebevorrichtung (21), die dergestalt ausgeführt ist, dass der Adapter (2) zwischen einem Bestückungszustand, der oberhalb des Wannenbades angeordnet ist und einem Reinigungszustand, bei dem der Adapter zumindest bereichsweise unter einer Wasseroberfläche angeordnet ist, verfahrbar ist. Die Anmeldung bezieht sich außerdem auf eine Bewegungsvorrichtung zur Verwendung in dem System zur Reinigung von medizinischen Instrumenten.

## Beschreibung

Das vorliegende Schutzrecht betrifft eine Bewegungsvorrichtung sowie ein System zur Reinigung von medizinischen Instrumenten. In besonderer Weise eignet sich dies als eine Zusatzvorrichtung für ein Ultraschall-Reinigungsgerät.

Bei der Reinigung von medizinischen Instrumenten durch niederfrequenten Ultraschall werden Fremdpartikel auf oder in medizinischen Instrumenten aufgrund spezifischer Ultraschallwirkmechanismen (wie Kavitation, Microstreaming, Jetbuilding etc.) gelöst.

Hierbei werden insbesondere chirurgische Instrumenten in einen Korb gelegt und anschließend in einer mit einer Flüssigkeit befüllten Wanne eines Ultraschall-Reinigungsgeräts eingebracht. Anschließend wird die Wanne mit Ultraschall beaufschlagt, so dass sich Fremdpartikel und Anhaftungen in kurzer Zeit lösen können. Optional wird dieser Prozess durch den Zusatz geeigneter Reinigungs- und/oder Desinfektionspräparate, die der Flüssigkeit zugegeben werden, unterstützt.

Eine weitere Anwendung ist aus dem Bereich der Laparoskopie bekannt. Hierbei können Fremdpartikel nicht nur an der Außenseite des Instruments, sondern auch in einem Lumen eines Instrumentenschafts des Instruments auftreten. Um hier eine reinigende Wirkung zu entfalten ist es bekannt, beispielsweise das distale Ende des Schaftes an einen Adapter anzuschließen, der es ermöglicht, Flüssigkeit vom proximalen zum distalen Ende des Schaftes zu ziehen, so dass neben der hierdurch eintretenden Spülwirkung auch die Wirkung des Ultraschalls innerhalb des Schaftlumens für die Reinigung nutzbar gemacht werden kann. Insbesondere bei komplexeren medizinischen Geräten, beispielsweise endoskopischen medizinischen Geräten, die mehrere Freiheitsgrade aufweisen, ist eine Reinigung sehr wichtig, um Infektionen bei nachfolgenden Operationen an Menschen oder Tieren zu vermeiden. Zunächst einmal ist es wichtig, dass sämtliche beweglichen Teile gereinigt und desinfiziert werden.

Hiervon ausgehend stellt sich die Aufgabe, eine Bewegungsvorrichtung bzw. ein System zur Reinigung von medizinischen Instrumenten bereitzustellen, das einerseits auch von ungeschultem Person leicht zu bedienen ist und außerdem eine sehr gründliche Reinigung medizinischer Instrumente ermöglicht.

Diese Aufgabe wird durch die Gegenstände der unabhängigen Ansprüche gelöst. Dies betrifft zunächst einmal eine Bewegungsvorrichtung, enthaltend einen Adapter zur Ankopplung mindestens eines medizinischen Instruments, wobei der Adapter mindestens ein mittels eines Antriebs bewegbares Kopplungselement umfasst, wobei das Kopplungselement so gestaltet ist, dass bei Ankopplung des medizinischen Geräts zumindest ein Bereich des medizinischen Geräts durch Bewegung des Kopplungselements in Bewegung versetzbar ist, wobei die Kraftübertragung zwischen dem Antrieb und dem Kopplungselement berührungslos erfolgt.

Durch die Bewegung des Kopplungselements und die dadurch verursachte Bewegung von beweglichen Teilen des medizinischen Instruments wird dieses "unter Bewegung" gereinigt, so dass sämtliche anhaftenden Partikel entfernt werden können und eine anschließende Desinfektion erfolgt. Insbesondere durch die berührungslose Kraftübertragung wird es hierbei notwendig, Teile des Antriebs bzw. des Abtriebs unterhalb einer Flüssigkeitsoberfläche des Ultraschallbads anzubringen. Dies ermöglicht beispielsweis eine bestmögliche Ausrichtung der medizinischen Geräte hin zu den Ultraschall aussendenden Wänden des Ultraschallbads.

Es ist besonders vorteilhaft, diese Bewegungsvorrichtung innerhalb eines Ultraschallbads anzubringen, das eine Steuerung nicht nur die Bewegung der medizinischen Instrumente sondern auch die Ultraschallintensität und einer Spülung (Druck- und/oder Saugspülung) des Instrumentenschafts ermöglicht. Außerdem ermöglicht insbesondere die berührungslose Kraftübertragung eine kinematische Entkopplung einzelner Freiheitsgrade bei der Bewegung der medizinischen Instrumente. Somit ist es möglich, ein genau vorgegebenes Programm zur Reinigung spezifischer medizinischer Instrumente abzurufen, bei dem jeder Bedienfreiheitsgrad des medizinischen Instruments nachgebildet werden kann. Nur auf diese Weise ergibt sich eine ganz klar nachvollziehbare und gut dokumentierbare Reinigung, die das Verbleiben kleinster Rückstände vermeiden hilft.

Das vorliegende Schutzrecht betrifft außerdem ein System zur Reinigung von medizinischen Instrumenten, vorzugsweise eine Bewegungsvorrichtung nach den oben genannten Merkmalen (wobei hier allerdings eine berührungslose Kraftübertragung nicht notwendig ist, diese kann auch mechanisch gekoppelt erfolgen). Für alle diese Systeme (also berührungslos oder mechanisch gekoppelt, beispielsweise durch Riemen, Zahnräder etc.) bietet es sich an, dass der Adapter zur Ankopplung mindestens eines medizinischen Instruments, der mittels eines Antriebs bewegbares Kopplungselement umfasst, wobei das Kopplungselement so gestaltbar ist, dass bei Ankopplung des medizinischen Instruments zumindest ein Bereich des medizinischen Instruments durch Bewegung des Kopplungselements in Bewegung versetzbar ist, weiterhin eine Schwenk- und/oder Hebevorrichtung aufweist, die dergestalt ausgeführt ist, dass der Adapter zwischen
a) einem Bestückungszustand, der oberhalb des Ultraschallbads bzw. eines Wannenbades angeordnet ist, und
b) einem Reinigungszustand, bei dem der Adapter zumindest bereichsweise unter einer Wasseroberfläche, beispielsweise eines Ultraschallreinigungsbads und/oder in einem Sprühnebel oder dergleichen angeordnet ist,
verfahrbar ist.

Auf diese Weise ist es für medizinisches Personal leicht möglich, die medizinischen Geräte an dem Adapter anzubringen und dann in einen Reinigungszustand zu verfahren, bei dem diese bestmöglich, beispielsweise ausgerichtet auf einen Sprühnebel oder eine Ultraschallquelle in einem Wasserbad, ausgerichtet sind.

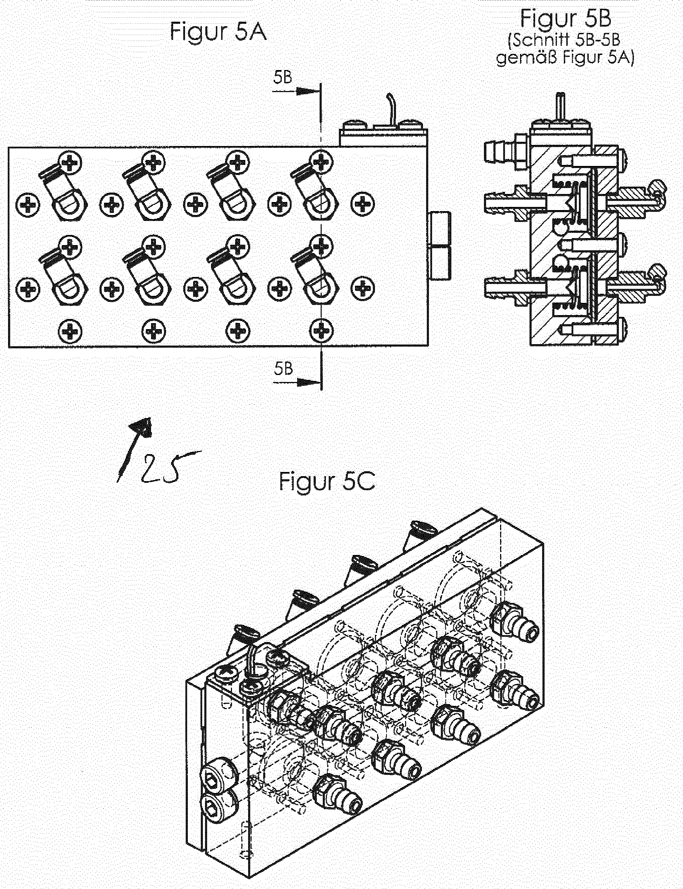

Im Folgenden werden weitere Ausbildungen der oben genannten Vorrichtung gezeigt. Es wird darauf hingewiesen, dass sämtliche Weiterbildungen auch miteinander kombinierbar sind, sofern dies nicht ausdrücklich als technisch inkompatibel genannt wird. Insbesondere bieten sich auch die Ausführungsformen der Schwenk- und/oder Hebevorrichtung für mechanisch kraftübertragende als auch berührungslose Antriebe der Kopplungselemente zum Antrieb der medizinischen Instrumente an. Insbesondere sind sämtliche Bewegungsvorrichtungen bzw. Systeme im Sinne dieser Schutzrechtsanmeldung kombinierbar mit einem Kanalwähler, der für eine Saug- und/oder Druckspülungsvorrichtung zur Spülung mindestens eines Lumens eines medizinischen Geräts vorgesehen ist. Dieser ist dadurch gekennzeichnet, dass er vorzugsweise pneumatisch betrieben und die beweglichen Teile beispielsweise aus Kunststoff bestehen. Der Kanalwähler (siehe auch das Beispiel aus Figuren 5a und 5c) eignet sich insbesondere für komplexe medizinische Instrumente, die mehrere Lumen haben bzw. für eine Vielzahl entsprechender medizinischer Instrumente. Vorteilhaft ist hierbei auch, dass geringe Totvolumina erzielbar sind und dass der Kanalwähler im Wesentlichen wartungsfrei ist, so dass auch langfristig gute Reinigungsergebnisse erzielt werden können. Es wird ausdrücklich darauf hingewiesen, dass sich die Anmelderin gesonderter Schutz auf diesen Kanalwähler gemäß den obigen Sätzen bzw. den Figuren vorbehält, beispielsweise durch eine Teilanmeldung.

Eine Ausführungsform der Bewegungsvorrichtung für medizinische Instrumente sieht vor, dass das mindestens eine Kopplungselement rotierbar ausgeführt ist. Hierbei ist dieses ankoppelbar an ein komplementäres Teil des zu reinigenden medizinischen Instruments und es wird hier durch Formschluss eine mechanische Kopplung zwischen dem anzutreibenden Teil des medizinischen Instruments sowie dem Kopplungselement hergestellt.

Es bietet sich auch an, dass mehrere Kopplungselemente in einem Adapter vorgesehen sind. Dies kann beispielsweise dergestalt sein, dass vier medizinische Instrumente vorgesehen sind zur Ankopplung an einen Adapter, wobei jedes der medizinischen Instrumente jeweils vier Kopplungselementen zugeordnet ist. Auf diese Weise können auch medizinische Instrumente mit mehreren Freiheitsgraden komplett (d.h. in allen Freiheitsgraden) bewegt und vorzugsweise zusätzlich nochmals durchspült werden, um so eine bestmögliche Reinigungswirkung zu erzielen.

Eine Ausführungsform sieht vor, dass die berührungslose Kraftübertragungseinrichtung als Magnetkupplung ausgeführt ist. Dies kann beispielsweise eine permanentmagnetische berührungslose Magnetkupplung sein, wie sie beispielsweise bei Magnetrührern für den Laborbedarf bekannt sind. Unter "berührungslos" sollen allerdings im Rahmen des vorliegenden Schutzrechts sämtliche berührungslose magnetische bzw. elektromagnetische bzw. induktive Verfahren fallen. Wichtig ist hier lediglich, dass durch eine Trennwand hindurch bzw. über einen Spalt hinweg mit einem bestimmten Abstand ein Antriebselement und ein Abtriebselement voneinander getrennt sind, um insbesondere eine Flüssigkeitsabdichtung zu erreichen.

Vorzugsweise sind hierfür der Antrieb und das Kopplungselement durch eine fluiddichte Trennschicht voneinander getrennt. Dies kann beispielsweise eine Kunststoffwand und/oder eine Kunststoffmembran sein. Auf diese Weise werden leicht zu reinigende und den Magnetfluss nicht störende Trennschichten vorgesehen.

Die Bewegungsvorrichtung wird, wie oben bereits erwähnt, vorzugsweise in einem mit Ultraschall beaufschlagbaren Bad angeordnet. Auf diese Weise kann eine sehr gute Reinigungswirkung erzielt werden, da neben der Bewegung der Instrumente in einem Wasserbad noch der Ultraschall zur zusätzlichen Reinigung herangezogen wird. Dies erfolgt in der Regel dadurch, dass in einem wannenförmigen Bad, auf dessen beispielsweise Außenseite Ultraschallerzeuger angebracht sind, ein Ultraschallfeld auf eine Flüssigkeit im Wannenband appliziert wird und die Bewegungsvorrichtung mit ihrem Adapter die medizinischen Instrumente so anordnet, dass diese medizinischen Instrumente zumindest bereichsweise unterhalb einer Flüssigkeitsoberfläche des Wannenbads sind.

Neben dieser Ausführungsform ist die Bewegungsvorrichtung nach dem Schutzrecht allerdings auch beispielsweise in einem Wasserstrahl oder in einem Wassersprühnebel platzierbar, um so auch eine Bewegung des medizinischen Instruments unter Flüssigkeitseinfluss zu erreichen.

Die medizinischen Instrumente, die hier zur Reinigung vorgesehen sind, sind vorzugsweise endoskopische Instrumente. Beispielsweise sind dies endoskopische Instrumente mit einem Koppelteil zur Ankopplung an den Adapter, einem sich an das Koppelteil anschließenden Instrumentenschaft und ein an dem dem Koppelteil abgewandten Ende des Instrumentenschafts angebrachten Operationsteil ausgeführt. Bei diesen Instrumenten ist es besonders wichtig, eine Reinigung durchzuführen, nicht nur im Bereich des Operationsteils sondern auch innerhalb des Instrumentenschafts, da sich bei ungenügender Reinigung hier infektiöses Material sammeln kann und die Patientensicherheit langfristig gefährden kann.

Oftmals weisen diese endoskopischen Instrumente mehrere unabhängig voneinander bewegbare Operationsteile auf, die von unterschiedlichen Kopplungselementen, unabhängig voneinander bewegbar sind.

Die unabhängige Bewegung einzelner Kopplungselemente ist ein Aspekt, für den die Anmelderin sich gesonderten Schutz vorbehält und diesen in einer möglicherweise späteren Teilanmeldung bzw. Nachanmeldung verfolgen möchte. Hierfür sind die folgenden Aspekte auch von Interesse:
Um eine vollständige und schonende Bewegung der Robotik-Instrumente (endoskopischer Instrumente) sicherzustellen und eine Überlastung auszuschließen, kann jede Achse eines Robotikinstruments (eines endoskopischen Instruments, siehe oben) von einem entsprechenden Aktor/Motor angetrieben werden. Jeder Aktor wird durch einen Mikroprozessor, der das Drehmoment des Aktors überwacht, geregelt. Die Regelschleife schließt aufgrund der Regel größer (beispielsweise Strom des Aktors), beispielsweise nur die Abtriebsseite des Aktors mit ein. Die magnetische Kupplung und ihr Einfluss auf Reibung und Bewegungsform sollten vorzugsweise durch ein Abgleichverfahren ermittelt werden. Das Drehmoment, welches für die vollständige und sichere Bewegung der Instrumente benötigt wird, plus die parasitären Drehmomente, ergeben sich aus der Reibung und sollten vom Aktor und Mikroprozessor (also der Steuereinheit) bereitgestellt und gesteuert werden. Aufgrund von Toleranzen in der Herstellung der Bewegungsvorrichtung sind die parasitären Einflüsse an jeder angetriebenen Achse möglicherweise unterschiedlich.

Hierzu ist es möglich, jede angetriebene Achse nach der Montage zu kalibrieren. Der Aktor treibt die Achse ohne Nutzlast, beispielsweise für 10 bis 100 Sekunden, z.B. 60 Sekunden an, während der Motorstrom des Aktors kontinuierlich gemessen wird. Der ermittelte Werte wird dann als Initialwert des Motorstroms für die Bewegung genutzt. Das zur vollständigen und sicheren Bewegung benötigte Drehmoment an dem Kopplungselement/den Robotikinstrumenten wird für jede Achse auf den Initialwert addiert. So ergibt sich für jede angetriebene Instrumentenachse eine immer gleichbleibende Drehmomentbelastung. Gegen Überlast gibt es beispielsweise in der Software einen Maximalwert für das erlaubte Drehmoment, welches die Grenze des Kraftschlusses der Magnetkopplung definiert. Des Weiteren ist auch ein mechanischer Schutz gegen Überlast der Instrumente gegeben, da auch die Magnetkupplung nur bis zu einem bestimmten Grenzdrehmoment hin eine Drehmomentenübertragung erreicht und danach durchrutscht.

Im Rahmen des vorliegenden Schutzrechts bzw. möglicher Teilanmeldungen ist es also möglich, nicht nur elektronisch eine Drehmomentbegrenzung vorzusehen sondern auch durch die Stärke der Magnetkupplung, beispielsweise durch die Auswahl der Stärke bzw. Anzahl von entsprechenden permanentmagnetischen Elementen in der Kraftübertragung.

Die oben beschriebenen Kalibriermöglichkeiten sind insbesondere für eine berührungslose Kraftübertragung gedacht. Mechanisch kann dies auf verschiedene Weise umgesetzt werden. So sieht eine Ausführungsform vor, dass ein antriebsseitiger Teil der Kraftübertragung ein rotierbares Antriebselement aufweist, das vorzugsweise zumindest bereichsweise permanentmagnetisches Material enthält und vorzugsweise axial verschieblich ist bezüglich anderer Teile des Antriebs. Dadurch wird es beispielsweise von den Magneten auf der Gegenseite stets an die Trennschicht (Membran) gezogen, dadurch werden auch Varianzen in magnetischer Übertragungskraft und Reibung verringert.

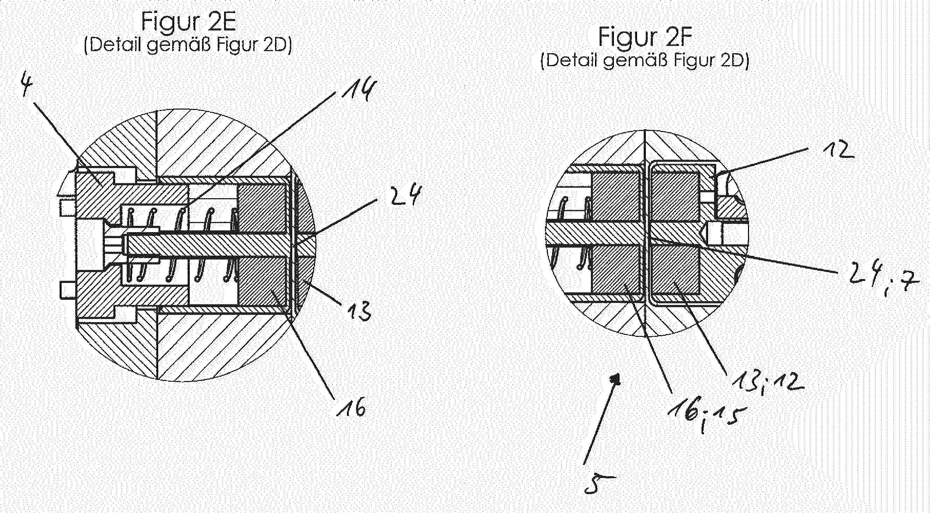

Eine weitere Ausführungsform sieht vor, dass ein abtriebsseitiger Teil der Kraftübertragung vorgesehen ist, in dem das Kopplungselement federnd gelagert ist. Bei einem solchen federnd gelagerten Kopplungselement/Mitnahmeteil spielt der Drehwinkel des entsprechenden Gegenstücks am medizinischen Instrument zunächst keine Rolle, es wird beim Einsetzen des Mitnahmeteils zunächst heruntergedrückt und rastet bei Drehung dann später ein (siehe beispielsweise Fig. 2E).

Eine weitere Ausführungsform sieht vor, dass bei dem abtriebsseitigen Teil der Kraftübertragung eine Druckfeder zwischen dem Kopplungselement und einem rotierbaren Abtriebselement angeordnet ist, wobei das Bewegungselement zumindest bereichsweise permanentmagnetisches Material enthält. Somit ist es also nicht notwendig, das Kupplungselement selber magnetisch auszuführen. Das Drehmoment wird in einem gesonderten Teil (das mit Permanentmagneten verbunden ist) aufgebracht, das Kopplungselement wird durch eine Feder hiermit verbunden.

Eine weitere Weiterbildung sieht vor, dass ein antriebsseitiger Teil und ein abtriebsseitiger Teil der Kraftübertragungseinrichtung jeweils mindestens einen Bereich aus permanentmagnetischem Material enthalten, wobei die Teile so aufeinander bezogen sind, dass die Drehung des antriebsseitigen Teils eine Drehung des abtriebsseitigen Teils nach sich zieht. Hierbei kann eine unterschiedliche Anzahl bzw. unterschiedliche Menge magnetischen Materials auf beiden Seiten vorgesehen sein. Beispielsweise ist es möglich, einzelne Permanentmagneten in Form eines Revolvers gegenüberliegend vorzusehen. Hierdurch wird zunächst eine gute Zentrierung erreicht, durch die Wahl der Anzahl und Menge bzw. Stärke des magnetischen Materials kann außerdem das Drehmoment (Rutschmoment) der Magnetkupplung vorgegeben werden.

Es ist auch, wie oben beschrieben, möglich, dass pro Kopplungselement jeweils ein Motor vorgesehen ist. Hiermit ist es möglich, eine unabhängige Bewegung und freie Programmierung sowohl des Bewegungsablaufs als auch von Schlupfdrehmomenten etc. einzustellen.

Eine Weiterbildung sieht vor, dass eine Saug- und/oder Druckspülungsvorrichtung zur Spülung mindestens eines Lumens des medizinischen Geräts vorgesehen ist. Hierdurch wird, ergänzend zu der mechanischen Bewegung, eine Spülung des Instrumentenschafts erreicht. Hierbei ist es auch möglich, in beide Richtungen eine Spülung zu erreichen, um so insbesondere bei Hinterschnitten besonders effizient reinigen zu können.

Hierbei ist auch eine Steuereinheit vorgesehen, welche nicht nur den Antrieb des Kopplungselements und somit der medizinischen Instrumente steuert sondern auch die Spülvorrichtung bzw. auch die Ultraschallbeaufschlagung. Auf diese Weise ist ein sehr individuelles Programm für einzelne medizinische Instrumenten abrufbar und dokumentierbar, so dass hier eine bestmögliche Reinigung erzielt und dokumentiert werden kann.

Eine Weiterbildung sieht vor, dass unterschiedliche Adapter in die Bewegungsvorrichtung integrierbar sind und auch untereinander austauschbar sind. Hierdurch wird gewährleistet, dass auch medizinische Instrumente unterschiedlicher Bauart bzw. unterschiedlicher Hersteller in einer einzigen Bewegungsvorrichtung gereinigt werden können. Hierdurch werden die Gesamtkosten für die Reinigung reduziert. Mit sämtlichen der oben genannten Bewegungsvorrichtungen ist eine Schwenk- und/oder Hebevorrichtung kombinierbar. Diese weist vorzugsweise eine Kinematik auf, die neben einem Senken und Heben auch eine Drehung erreicht. Hierdurch wird es möglich, dass zum einen in einem für den Bediener günstigen Zustand die Adapter bestückbar sind und in einem Reinigungszustand die medizinischen Instrumente bestmöglich im Wasserbad bzw. im ultraschallbeaufschlagten Wasserbad angeordnet werden können. Hierbei ist es besonders Vorteil, dass die Bestückung durch das medizinische Personal so erfolgt, dass von einer stehenden Bedienperson aus gesehen eine frontale Ankopplung der medizinischen Instrumente an den Adapter möglich ist und hier keine "Überkopfarbeiten" etc. nötig sind. Für den Reinigungszustand ist es besonders vorteilhaft, wenn dieser so ausgerichtet ist, dass eine bestmögliche Beaufschlagung mit Ultraschall erfolgen kann, d.h. keine störenden Körbe oder Gestelle zwischen der Wand des Ultraschallbades und dem medizinischen Instrument liegen.

Weitere Weiterbildungen werden in der speziellen Beschreibung genannt.

Weitere Details werden nun anhand von Figuren erläutert. Es zeigen:
- Fig. 1: ein System zur Reinigung von medizinischen Instrumenten, enthaltend eine Bewegungsvorrichtung für medizinische Instrumente,
- Fig. 2a: das System aus Fig. 1 in einem Bestückungszustand für medizinische Instrumente (siehe links) sowie im Reinigungszustand (siehe rechts),
- Fig. 2b: eine erste Ausführungsform eines Adapters einer erfindungsgemäßen Bewegungsvorrichtung mit schwebend davor dargestellten medizinischen Instrumenten zur Reinigung;
- Fig. 2c: eine Draufsicht eines Adapters zur Ankopplung medizinischer Instrumente gemäß Fig. 2b (jetzt ohne schwebend davor dargestellte medizinische Instrumente),
- Fig. 2d: ein Schnitt gemäß 2D-2D nach Fig. 2c,
- Fig. 2e: ein Detail gemäß Fig. 2d,
- Fig. 2f: ein Detail gemäß Fig. 2d,
- Fig. 2g: ein Schnitt gemäß 2G-2G nach Fig. 2c,
- Fig. 3a: eine weitere Variante eines Systems, nun mit einem anderen Adapter als in Fig. 2a, wiederum im Bestückungszustand (siehe links) und im Reinigungszustand (siehe rechts),
- Fig. 3b: zwei Ansichten des Adapters/ der Bewegungsvorrichtung nach Fig. 3a mit davor schwebend angeordneten medizinischen Instrumenten,
- Fig. 3c: eine Ansicht eines Adapters nach Fig. 3a (ohne davor schwebende medizinische Instrumente,
- Fig. 3d: ein Schnitt gemäß 3D-3D nach Fig. 3c,
- Fig. 4a: eine Schwenk- und/oder Hebevorrichtung zur Ankopplung einer Bewegungsvorrichtung bzw. eines Adapters,
- Fig. 4b: ein Schnitt gemäß 4B-4B nach Fig. 4a,
- Fign. 4c bis 4e: Schnitte der Schwenk- und/oder Hebevorrichtung nach Fig. 4a,
- Fign. 4f bis 4h: drei verschiedene Bewegungszustände eines Systems sowie einer Bewegungsvorrichtung in der Seitenansicht sowie
- Fign. 5a bis 5c: Ansichten und Einschnitte eines Kanalwählers zur Verwendung in einer Druck- und/oder Saugvorrichtung zum Spülen medizinischer Instrumente.

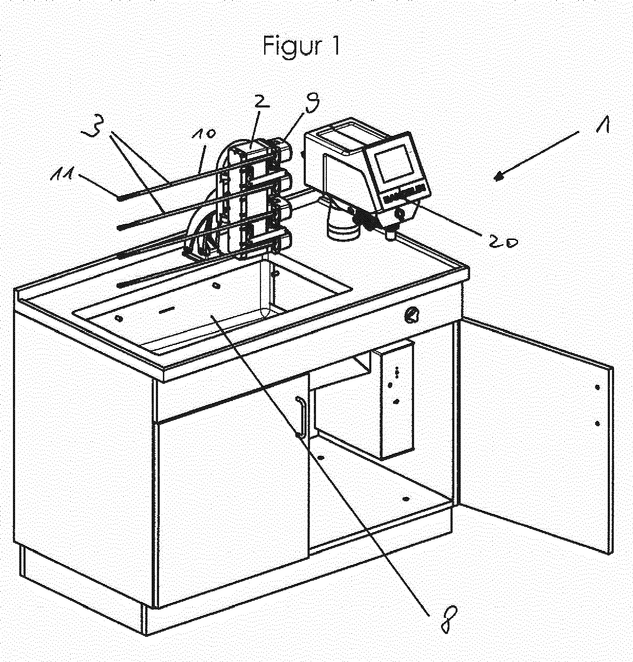

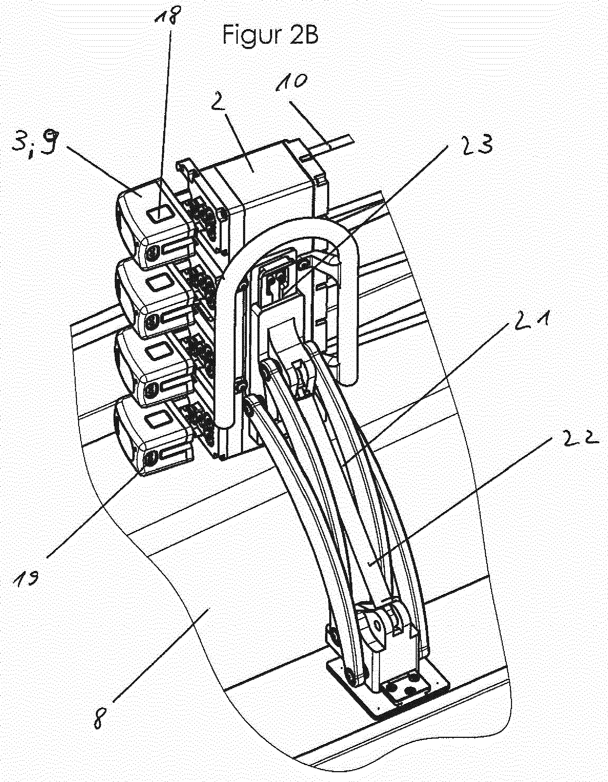

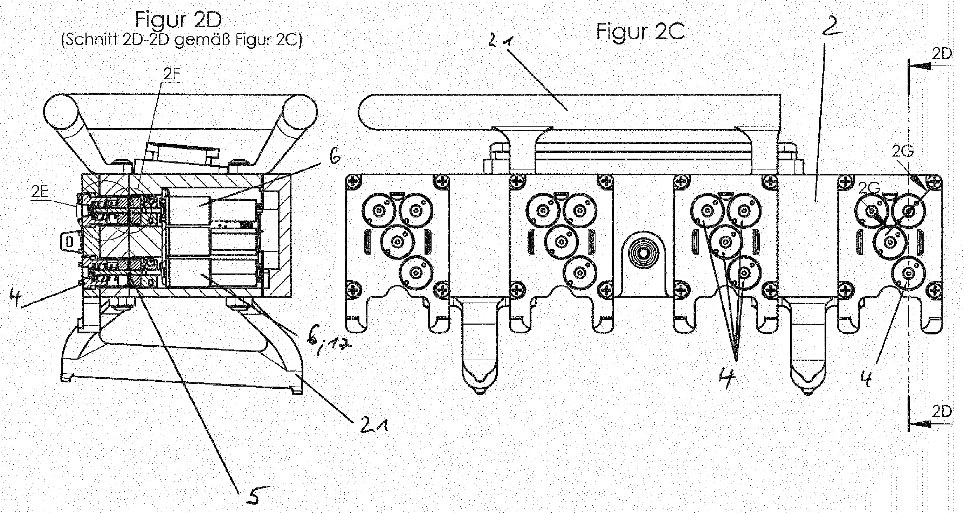

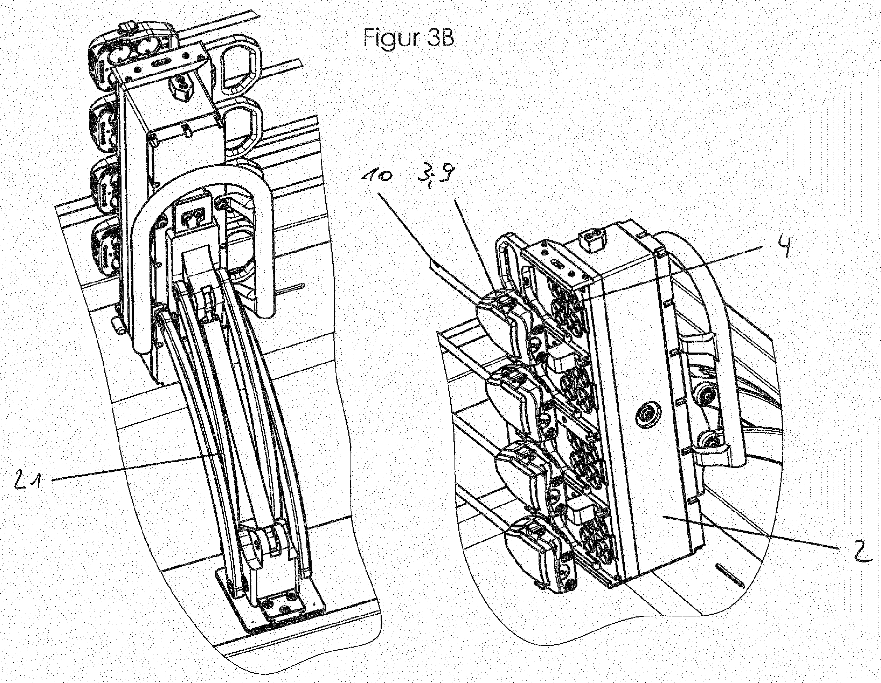

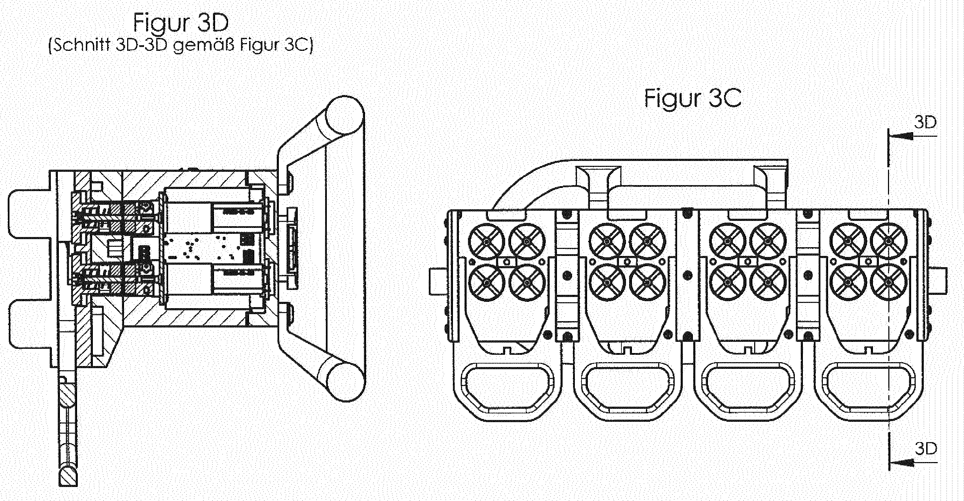

Fig. 1 zeigt ein System, enthaltend eine Bewegungsvorrichtung 1, enthaltend einen Adapter 2 zur Ankopplung mindestens eines medizinischen Instruments 3. Der Adapter 2 enthält mindestens ein mittels eines Antriebs bewegbares Kopplungselement 4 (siehe hierzu Fign. 2b ff. bzw. Fign. 3b ff.), wobei das Kopplungselement so gestaltet ist, dass bei Ankopplung des medizinischen Geräts 3 zumindest ein Bereich des medizinischen Geräts durch Bewegung des Kopplungselements in Bewegung versetzbar ist. Die Kraftübertragung zwischen dem Antrieb und dem Kopplungselement erfolgt berührungslos, Details hierzu sind beispielsweise in Fign. 2d bis 2g beispielhaft ausgeführt, in denen rotierbare Kopplungselemente gezeigt werden. In allen Beispielen sind jeweils mehrere Kopplungselemente in einem Adapter vorgesehen und die berührungslose Kraftübertragungseinrichtung ist als Magnetkupplung ausgeführt. Dies ist allerdings nicht zwingend, alternativ können auch andere berührungslose Verfahren bzw. unterschiedlichen Zahlen von Kopplungselementen vorgesehen (siehe Beschreibungseinleitung). Ebenfalls in Fig. 1 ist eine Steuereinheit 20 zu sehen. Diese Steuereinheit bewirkt zum einen die Steuerung des Antriebs sämtlicher Kopplungselemente. Außerdem wird durch diese Steuereinheit eine Spülvorrichtung zur Saug- und/oder Druckspülung der medizinischen Instrumente 3 gewährleistet. Schließlich wird auch eine Ultraschallvorrichtung gesteuert, die ein Wasserbad 8 mit Ultraschall beschallt. Hierzu sind an der Außenseite des Wasserbads, beispielsweise im Bodenbereich und/oder an den Seitenwänden Schwingsysteme zur Ultraschallerzeugung angebracht. Die Steuereinheit 20 hat außerdem vorgefertigte Programme für gezielte Beschallungs-/Spülungs-/Antriebssteuerung spezifischer medizinischer Instrumente. Außerdem können zur Dokumentation von Reinigungsvorgängen entsprechende Protokolle erstellt und gegebenenfalls per Datenleitung überspielt bzw. gespeichert werden.

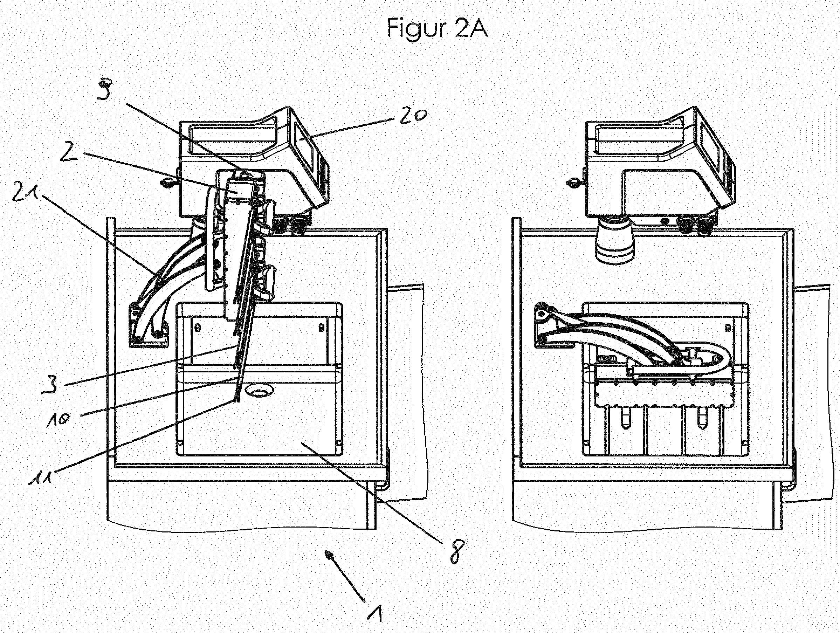

Fig. 2a zeigt linksseitig ein System 1 mit einem Ultraschallbad 8 (in diesem Zustand nicht mit Wasser befüllt) sowie einer darüber angeordneten Schwenk- und/oder Hebevorrichtung mit angekoppeltem Adapter 2, an den medizinische Instrumente 3 angekoppelt sind. Die medizinischen Instrumente 3 weisen einen Instrumentenschaft 10 auf. An diesen Instrumentenschaft 10 schließen sich einerseits die Koppelteile 9 (siehe Fig. 2b an, in diesen Koppelteilen sind Mechaniken zur Bewegung eines Operationsteils 11 am anderen Ende des Instrumentenschafts 10 angebracht). Diese Koppelteile haben in der Regel mehrere Mechaniken, die an entsprechende Koppelteile angekoppelt werden, um die Mechaniken zu bewegen und dadurch sämtliche Stellen bei einer Reinigung gut zu erreichen (Details hierzu weiter unten).

In Fig. 2a ist nun gut zu sehen, dass durch eine kombinierte Schwenk- und Hebebewegung der Schwenk- und/oder Hebevorrichtung 21 der Adapter mit den angekoppelten medizinischen Instrumenten bestückt werden kann. In der in Fig. 2a links gezeigten Ausführungsform kann eine Bedienperson, die vor dem System (siehe Fig. 1) steht, einfach die Bestückung des Adapters 2 mit medizinischen Instrumenten 3 vornehmen. Außerdem kann auch die Steuereinheit 20 in dieser Position leicht bedient werden. Sobald die Bestückung mit den medizinischen Instrumenten erfolgt ist, kann die Schwenk- und/oder Hebevorrichtung in die in Fig. 2a rechts gezeigte Position verbracht werden.

Fig. 2b zeigt eine weitere Ansicht der Darstellung aus Fig. 2a (linke Seite), bei der die medizinischen Instrumente 3 schwebend vor dem Adapter 2 dargestellt sind. Hierdurch wird sichtbar, dass eine Ankopplung (also eine Fixierung, vorzugsweise durch Einschnappen) der Koppelteile 9 der medizinischen Instrumente 3 an den Adapter 2 erfolgt. Zusätzlich können auch zur Spülung von mindestens einem Lumen 19 an dem Koppelteil eine Druck- und/oder Spülvorrichtung angebracht werden, die den Instrumentenschaft 10 des medizinischen Instruments spült.

Die in Fig. 2b gezeigte Schwenk- und Hebevorrichtung zeichnet sich durch Stabilität und gute Bedienung aus. Es ist möglich, die Vorrichtung 21 in den extremen Positionen zu rasten (d.h. in dem Reinigungszustand (wie in Fig. 2a rechts) oder auch im Bestückungszustand (Fig. 2a links bzw. Fig. 2b)). Um eine unnötig hohe Geschwindigkeit beim Schwenken/Heben zu vermeiden ist eine Gasdruckfeder 22 vorgesehen. Auf Details des Schwenkmechanismus wird weiter unten nochmals eingegangen. Außerdem weist die Schwenk- und Hebevorrichtung einen U-förmigen Hebel auf, so dass der Adapter 2 nicht unmittelbar angefasst werden muss von einer Bedienperson. Außerdem ist zu sehen, dass der Adapter 2 mit der Schwenk- und Hebevorrichtung 21 durch einen Rastmechanismus 23 verbindbar ist, um so unterschiedliche Adapter ankoppeln zu können.

Fig. 2c zeigt eine Ansicht des in Fig. 2b gezeigten Adapters 2 (ohne davor liegende medizinische Instrumente 3). Zu sehen ist hier außerdem der U-förmige Haltegriff des Schwenk- und/oder Hebemechanismus 21. Der Adapter aus Fig. 2c ist zur Ankopplung von vier medizinischen Instrumenten 3 geeignet. Jedes dieser medizinischen Instrumente weist vier Mechaniken auf, die über entsprechende Kopplungselemente 4 bewegbar sind. Jede Mechanik sorgt für eine Bewegung am Ende des Instrumentenschafts, also am Operationsteil 11 eines medizinischen Instruments (siehe Fig. 2a links). Die Steuerung der einzelnen Kopplungselemente 4 kann völlig unabhängig voneinander durch die Steuereinheit 20 erfolgen. Es gibt somit keine kinematischen Zwangsbewegungen zwischen einzelnen Kopplungselementen, jedes Kopplungselement ist vom Bewegungszyklus her frei verfahrbar. Vorteilhaft ist außerdem, dass individuell für jedes Kopplungselement entweder elektronisch oder mechanisch ein Maximaldrehmoment vorgesehen werden kann, so dass eine Beschädigung am Operationsteil 11 des medizinischen Instruments ausgeschlossen wird. Selbstverständlich ist es aber auch möglich, durch einen einzelnen Antrieb sowohl die Kopplungselemente eines einzelnen medizinischen Instruments gemeinsam oder auch für alle medizinischen Instrumente gemeinsam zu bewegen.

Fig. 2d zeigt einen Schnitt gemäß 2D-2D aus Fig. 2c. In diesem Schnitt werden zwei Kopplungselemente im Schnitt dargestellt, die nach Maßgabe von zwei Antrieben 6 angetrieben werden. Zwischen dem Antrieb 6 und dem Kopplungselement 4 ist eine Kraftübertragung in Form einer Magnetkupplung 5 vorgesehen. Auf Details dieser Magnetkupplung 5 wird im Folgenden nochmals eingegangen.

Fig. 2e zeigt ein federnd gelagertes Kopplungsteil, der Drehwinkel des entsprechenden Gegenstücks an der Mechanik des Koppelteils 9 des medizinischen Instruments 3 spielt somit keine Rolle, da beim Einsetzen des Koppelelements 9 in den Adapter 2 das Mitnahmeteil zunächst heruntergedrückt wird und dann bei der Rastung in die entsprechende Mechanik des medizinischen Instruments einrastet. In der Fig. 2e ist im Detail besonders gut zu sehen, dass permanentmagnetisches Material 6 (beispielsweise zylinderförmige Magnete) in einem entsprechenden Abtriebsteil vorgesehen sind, das rotatorisch durch den Antrieb 6 (z.B. Motor 17, siehe hierzu weiter unten) angetrieben wird. Außerdem ist eine Druckfeder 14 vorgesehen, die zwischen Kopplungselement 4 und dem Teil, in dem das permanentmagnetische Material 16 untergebracht, eine axiale Verschiebbarkeit ermöglicht. Radial ist eine Zwangsführung zwischen diesen beiden gezeigt, so dass ein Drehen des permanentmagnetischen Materials 16 zu einem Drehen des Kopplungselements 4 führt.

Fig. 2f zeigt ein Detail gemäß Fig. 2d. Hier ist gut zu sehen, dass bei der Magnetkupplung 5 das Drehmoment der Motoren über Dauermagneten durch eine geschlossene Membran von dem wasserdichten Innenraum des Antriebs (die elektrischen Teile des Antriebs 16 im Adapter sind somit wasserdicht gekapselt) nach außen übertragen werden. Gemäß dem Detail nach Fig. 2f wird durch kugelförmige Aufsetzer auf beiden Magnetteilen die Reibung auf der Membran (z.B. einer Kunststoffwand 7 bzw. Kunststoffmembran) minimiert. Zu sehen ist also, dass durch permanentmagnetisches Material (Dauermagnete 13; siehe Antriebselement 12) rechtzeitig der Antrieb durch den Antrieb 6 erfolgt, der Abtrieb erfolgt linksseitig dadurch, dass das permanentmagnetische Material 16 (siehe Abtriebselement 15) entsprechend dem permanentmagnetischen Material 13 gedreht wird.

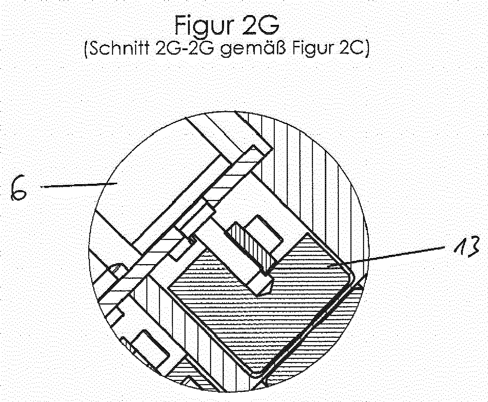

Fig. 2g zeigt einen weiteren Schnitt (siehe Schnitt 2G-2G gemäß Fig. 2c). Hierin ist gezeigt, dass die Motorachse des Antriebs 6 in axialer Richtung frei ist: da bei einer feste Positionierung des motorseitigen Magnetteils (mit permanentmagnetischem Material 13) der Abstand zur Membran variieren könnte, ist in dieser Ausführungsform eine Verbindung des Motors mechanisch nur in radialer Richtung gegeben, in axialer Richtung ist ein Bewegungsspielraum vorgesehen. Dadurch wird es von den Magneten auf der Gegenseite stets an die Membran gezogen, auf diese Weise werden Varianzen in magnetischer Übertragungskraft und Reibung verringert.

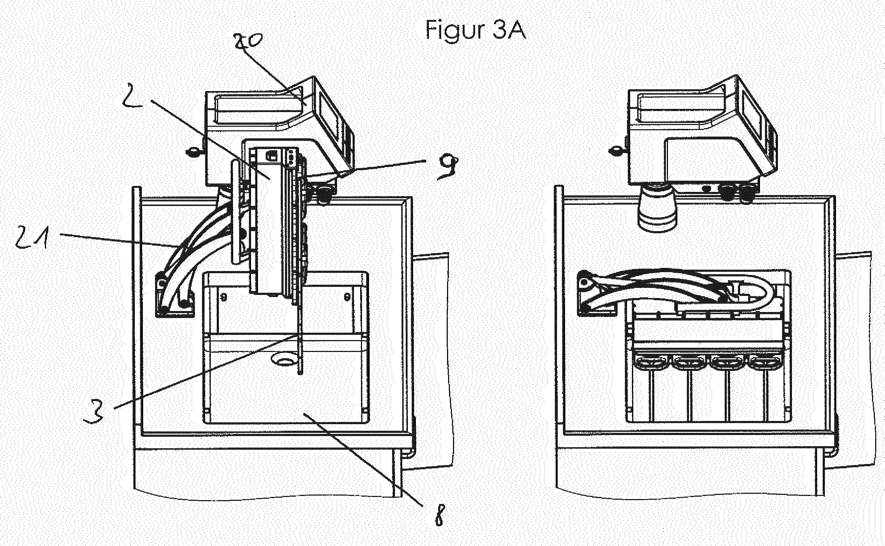

Fig. 3a zeigt eine weitere Ausführungsform eines Systems 1, wobei hier der Adapter 2 unterschiedlich ausgebildet ist. Der Unterschied besteht darin, dass eine andere Art medizinischer Instrumente 3 ankoppelbar ist. Im Wesentlichen ist der Unterschied zwischen Fig. 2a und 2b darin zu sehen, dass die Koppelteile 9 nun im Reinigungszustand (siehe Fig. 3a rechts) zum Boden der Wanne hin zeigen, während diese in der Ausführungsform nach Fig. 2a im Wesentlichen zu einer Wand hin orientiert sind.

Dies wird durch die Details nach Fign. 3b bis 3d nochmals deutlich, wobei zur Vermeidung von Wiederholungen auf die entsprechende Beschreibung der Fign. 2a bis 2f verwiesen wird.

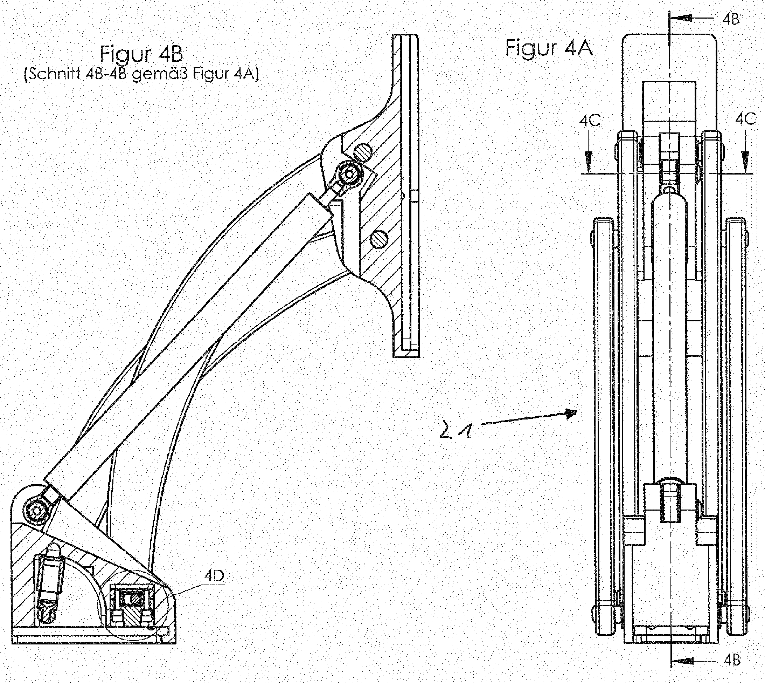

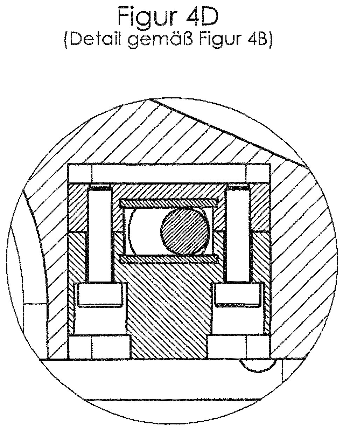

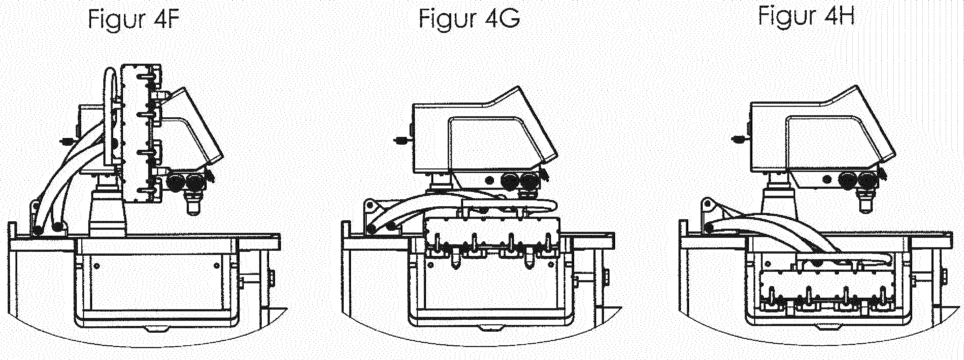

Fig. 4a zeigt nun eine Draufsicht einer erfindungsgemäßen Schwenk- und Hebevorrichtung 21 mit entsprechenden Hebeln sowie einer Gasdruckfeder 22. Hierbei sind auch Federbolzen vorgesehen, damit die Schwenk- und Hebevorrichtung auf einer bestimmten Position eine für den Anwender spürbare Rastposition hat, befindet sich auf einer mit den Armen des Schwenkhebels verbundenen Welle ein gefederter Bolzen, der auf dieser Position in einer Tasche im Gegenstück einrastet. Details hierzu sind in den weiteren Darstellungen (Fign. 4b bis 4e) gezeigt. In Fig. 4b ist ein Sperrbolzen gezeigt, der folgende Funktion hat: die Schwenk- und Hebevorrichtung 21 soll nicht von ihrer Aufnahme entfernbar sein, wenn diese nicht in der obersten Position (Bestückungszustand) steht. Deswegen sitzt ein Bolzen exzentrisch gelagert auf einer mit den Armen verbundenen Welle. Erfolgt eine Bewegung in den Reinigungszustand, taucht der Bolzen in eine entsprechende Bohrung und blockiert.

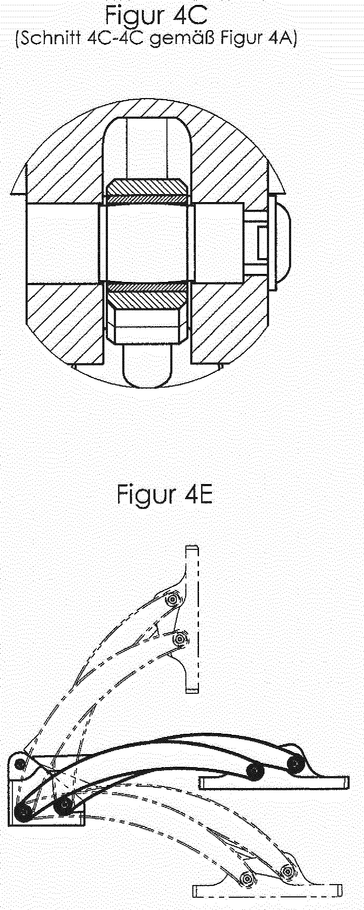

Fig. 4c zeigt Details einer Lagerung der Gasdruckfeder (der Begriff Gasdruckfeder schließt auch so genannte "Gaszugfedern" ein; wichtig ist lediglich, dass durch ein kompressibles Material hier eine Federung/Dämpfung der Bewegung möglich ist). Die Gasdruckfeder ist, wie in Fig. 4c zu sehen, auf einer leichtballigen Welle gelagert. So kann sie keine Torsionskräfte erfahren, welche sie unsachgemäß belasten und zu Undichtigkeit führen könnten, was einen Abfall der Kraft der Gasdruckfeder zur Folge hätte.

Fig. 4e zeigt nochmals Details zur Bewegungskinematik der Schwenk- und/oder Hebevorrichtung 21. Hierbei ist in der oberen schraffierten Stellung der Bestückungszustand gezeigt (siehe Fig. 2a bzw. Fig. 3a, jeweils links), sowie in der unteren schraffierten Stellung der Reinigungszustand (siehe Fig. 2a bzw. Fig. 3a, jeweils rechte Position). Zu Zwecken der Verdeutlichung ist der Adapter 2 hier nicht gezeigt. Hier ist nochmals gut zu sehen, dass im Bestückungszustand durch die senkrechte Position eine leicht frontale Erreichbarkeit durch eine Bedienperson möglich ist, im Reinigungszustand ist eine gute Ausrichtung zu einem flachen Wannenboden möglich. Aus der Kinematik ist ersichtlich, dass es sich hierbei um eine Schwenk- und Hebekinematik handelt. Hierdurch wird erreicht, dass der Schwenkradius relativ klein gehalten wird und somit eine bestmögliche Raumausnutzung erreicht wird, d.h., dass das notwendige Volumen im Ultraschallbad klein gehalten werden kann, wodurch eine noch effizientere Ultraschallbeschallung möglich wird.

Im Folgenden wird mit Bezug auf die Figuren 4f und 4g nochmals der Bewegungszustand der Schwenk- und Hebevorrichtung 21 mit angekoppeltem Adapter (die Lagen entsprechen hier den in Fig. 4e gezeigten Zuständen) gezeigt. Hier wird mit anderen Worten auch nochmals auf Aspekte dieser Schwenk- und Hebevorrichtung 21 eingegangen:
Aufgrund des Bedarfs an einem optimalen und einfachen Handling und der vorgegebenen geometrischen Gegebenheiten sollte also eine Möglichkeit gefunden werden, einen Kasten zunächst linear und ab einem bestimmten Punkt rotatorisch zu bewegen. Dies wird mittels einer Zwei-Arm-Führung umgesetzt, welche außerdem durch eine Gaszugfeder (bzw. Gasdruckfeder) unterstützt wird. Weiterhin gewährleistet das System eine einfache Demontierbarkeit der Komponenten und implementierte Sicherung gegen eine falsche Bedienung.

Zunächst einmal wird auf grundsätzliche Bewegungsformen eingegangen, die in den Figuren 4e bis 4h besonders gut zu sehen sind. Sind (siehe Fig. 4e) zwei gleich lange Arme gegeben, deren Bewegungsachsen auf beiden Seiten in ihren Lagerungsteilen parallel und in gleicher Distanz zueinander montiert sind, bewegen diese Lagerungsteile je nach ihrer Stellung sich zueinander entweder parallel oder rotatorisch. In der Stellung, in der sich alle vier Achsen auf einer Linie befinden, kann die Bewegung von der einen in die andere Form umschlagen (siehe Fig. 4e). Durch bestimmte Anschläge und Hindernisse werden die Arme nun daran gehindert, sich in der nicht gewünschten Form zu bewegen. Außerdem ist eine externe Kraft vorgesehen, welche die Arme in die gewünschte Bewegungsform (d.h. Bestückungszustand oder Reinigungszustand) drängt. Durch eine geometrische Vorgabe der Anschläge/Hindernisse wird unter Umständen in der Praxis kein langfristig belastbares Ergebnis erzielt, da durch Fertigungstoleranzen und elastische Verformung der Teile ein Verfallen in die ungewünschte Bewegungsform nicht gänzlich ausgeschlossen werden kann. Durch die externe Kraft, welche die Arme in die gewünschte Bewegungsform drängt, wird eine saubere Bewegung der Arme in der gewünschten Form ermöglicht, um ein gewaltsames Drängen des Anwenders in die nicht gewünschte Form zu unterbinden.

Im Folgenden wird kurz auf die Funktion der Gasdruckfeder/Gaszugfeder eingegangen.

In der beschriebenen Mechanik ist außerdem eine Gaszugfeder integriert. Diese erfüllt im Wesentlichen zwei Funktionen: (1) Entlastung des Anwenders beim Bewegen des Arms sowie (2) Drängen des Arms in die gewünschte Bewegungsform. Durch ihre Zugkraft wird die Gaszugfeder bei entsprechender Montage entgegen der Gewichtskraft einer an der Mechanik befestigten Last belastet. Möchte die Bedienperson diese Last an der Mechanik bewegen, so wirkt die Gaszugfeder unterstützend, so dass der Anwender nicht der kompletten Gewichtskraft der Last entgegenwirken muss. Das kann so weit gehen, dass auf einen Impuls des Anwenders hin die Last selbständig bewegt wird. Weiterhin kann die Gaszugfeder, wie oben beschrieben, den Arm in seiner Bewegungsform halten und auch bis zu einem gewissen Grad den Arm aus einer ungewünschten in die gewünschte Form drängen. Hierbei ist die Position der Achsen der Feder auf beiden Lagerungsteilen zu beachten. Sie muss abgestimmt sein mit der Federkraft, der Gewichtskraft der Last und der Geometrie der Mechanik.

Bei dem vorgesehenen Teil gemäß den Figuren, die rein beispielhaft zu verstehen sind, wurde ein spiegelsymmetrischer Aufbau mit einer mittig positionierten Gaszugfeder und vier Armteilen gewählt. Jeweils zwei dieser Armteile liegen auf denselben Achsen, um zu vermeiden, dass Kräfte und Momente in seitlicher Richtung auftreten. Hierzu sind die Armpaare so miteinander verbunden, dass sie relativ zu ihrer Lagerungswelle und somit zueinander nicht verdrehen können. So wird einer seitlichen Verdrehung des Arms weiter entgegengewirkt. Die Geometrie der Schwenk- und/oder Hebevorrichtung 21 und insbesondere die Positionierung und Kraft der Gaszugfeder/Gasdruckfeder 22 wurden so gewählt, dass ein Kräfteverlauf entsteht, bei welchem die Last in der untersten Position (Reinigungszustand) diese anhält, auf halber Höhe gleichen sich Last und Zugkraft der Gasdruckfeder aus, so dass die Vorrichtung 21 ohne weitere äußere Belastung in dieser Position verbleibt. Bewegt man die Vorrichtung 21 höher, überwiegt die Zugkraft, und der Schwenkmechanismus wird automatisch in den Bestückungszustand (oberste Position) bewegt. Die eingesetzte Gasdruckfeder 22 ist geschwindigkeitsbegrenzt, um Belastungen der Vorrichtung 21 und Gefahren für den Anwender zu vermeiden.

Wie bereits oben beschrieben, ist die Lagerungswelle der Gaszugfeder/Gasdruckfeder leicht ballig, so dass diese keine seitlich wirkenden Momente erfährt, welche im schlimmsten Fall zu Undichtigkeiten des Gasdruckzylinders führt. Die Vorrichtung 21 kann mittels beispielsweise schienenförmiger Fixiervorrichtungen an einem Bedientisch (siehe Fig. 1) fixiert werden, außerdem können unterschiedliche Adapter 2 (siehe ebenfalls Fig. 1) an der Vorrichtung positioniert werden. Befindet sich die Vorrichtung 21 nicht auf ihrer obersten Position (z.B. Bestückungszustand), so soll keine Demontage von der Halterung möglich sein. Hierfür besitzt mindestens eine der Verbindungswellen der Armteile einen exzentrischen Teil. Auf diesem läuft ein Bolzen, welcher beim Herunterbewegen nach unten fährt und in eine Bohrung taucht. Will der Anwender nun die Vorrichtung 21 aus ihrer Halterung ziehen, blockiert der Bolzen. Die Sperrbohrung befindet sich beispielsweise auf einem Blech, welches auf das Halterungsteil der Vorrichtung 21 aufgeschraubt wird. Unter der Zunge des Blechs mit der Sperrbohrung befindet sich ein Hohlraum. Drückt der Anwender die Vorrichtung 21 nach unten, ohne ihn komplett aufgeschoben zu haben, so dass der Bolzen nicht in die Bohrung tauchen kann, so wird die Blechzunge nach unten gedrückt und die Vorrichtung ansonsten nicht beschädigt.

Die Materialwahl für die Vorrichtung 21 ist bedarfsgerecht auszusuchen. Die Armteile können beispielsweise aus mit blauem Kunststoff umgossenen Metallgerüsten bestehen. Hierdurch wird zum einen die benötigte Stabilität gewährleistet, außerdem wird eine leichte Reinigung ermöglicht. Durch die gebogene Form (siehe Figuren, die diese gebogene Form in der Seitansicht zeigt) wird vermieden, dass das Armteil mit der Kante einer Wanne eines Ultraschallbads 8 (siehe Fig. 1) kollidiert.

Die Figuren 5a bis 5c zeigen Details eines Kanalwählers 25, der in einem System 1, insbesondere zur Anwendung in einer Saug- und/oder Druckspülvorrichtung zum Spülen eines Lumens eines medizinischen Instrumentes 3. Auf die Funktionsweise eines entsprechenden Kanalwählers, für den auch selbständiger Schutz erwünscht ist, wird im einleitenden Teil der Beschreibung bereits eingegangen.

Die Erfindung bezieht sich, inter alia, auf die folgenden Aspekte:
1. Bewegungsvorrichtung (1), enthaltend
   - einen Adapter (2) zur Ankopplung mindestens eines medizinischen Instruments (3), wobei
   - der Adapter (2) mindestens ein mittels eines Antriebs bewegbares Kopplungselement (4) umfasst, wobei das Kopplungselement so gestaltet ist, dass bei Ankopplung des medizinischen Geräts zumindest ein Bereich des medizinischen Geräts durch Bewegung des Kopplungselements in Bewegung versetzbar ist, wobei
   - die Kraftübertragung zwischen dem Antrieb und dem Kopplungselement berührungslos erfolgt.
2. Bewegungsvorrichtung nach Aspekt 1, dadurch gekennzeichnet, dass das Kopplungselement (4) rotierbar ausgeführt ist.
3. Bewegungsvorrichtung nach einem der vorhergehenden Aspekte, dadurch gekennzeichnet, dass mehrere Kopplungselemente (4) in einem Adapter vorgesehen sind.
4. Bewegungsvorrichtung nach einem der vorhergehenden Aspekte, dadurch gekennzeichnet, dass die berührungslose Kraftübertragungseinrichtung als Magnetkupplung (5) ausgeführt ist.
5. Bewegungsvorrichtung nach einem der vorhergehenden Aspekte, dadurch gekennzeichnet, dass ein Antrieb (6) und das Kopplungselement (4) durch eine fluiddichte Trennschicht voneinander getrennt sind.
6. Bewegungsvorrichtung nach Aspekt 5, dadurch gekennzeichnet, dass die Trennschicht als Kunststoffwand und/oder als Kunststoffmembran ausgeführt ist.
7. Bewegungsvorrichtung nach einem der vorhergehenden Aspekte, dadurch gekennzeichnet, dass diese so ausgeführt ist, dass sie in einem Wasserbad, einem Ultraschallbad (8), einem Wasserstrahl und/oder in einem Wassersprühnebel platzierbar ist.
8. Bewegungsvorrichtung nach Aspekt 7, dadurch gekennzeichnet, dass das Wasserbad mit Ultraschall beaufschlagbar ist.
9. Bewegungsvorrichtung nach einem der vorhergehenden Aspekte, dadurch gekennzeichnet, dass das mindestens eine medizinische Instrument (3) ein endoskopisches Instrument mit mindestens einem beweglichen Teil ist, das nach Maßgabe eines Kopplungselements bewegbar ist.
10. Bewegungsvorrichtung nach Aspekt 9, dadurch gekennzeichnet, dass das medizinische Gerät (3) ein endoskopisches Instrument mit einem Koppelteil (9) zur Ankopplung an den Adapter, einem sich an das Koppelteil anschließenden Instrumentenschaft (10) und ein an dem dem Koppelteil abgewandten Ende des Instrumentenschafts angebrachten Operationsteil (11) ausgeführt ist.
11. Bewegungsvorrichtung nach Aspekt 10, dadurch gekennzeichnet, dass mehrere unabhängig voneinander bewegbare Operationsteile (11) vorgesehen sind, die von unterschiedlichen Kopplungselementen (4) unabhängig voneinander bewegbar sind.
12. Bewegungsvorrichtung nach einem der vorhergehenden Aspekte, dadurch gekennzeichnet, dass ein antriebsseitiger Teil der Kraftübertragung ein rotierbares Antriebselement (12) aufweist, das vorzugsweise zumindest bereichsweise permanentmagnetisches Material (13) enthält und vorzugsweise axial verschieblich ist bezüglich anderer Teile des Antriebs.
13. Bewegungsvorrichtung nach einem der vorhergehenden Aspekte, dadurch gekennzeichnet, dass ein abtriebsseitiger Teil der Kraftübertragung vorgesehen ist, in dem das Kopplungselement (4) federnd gelagert ist.
14. Bewegungsvorrichtung nach einem der vorhergehenden Aspekte, dadurch gekennzeichnet, dass bei dem abtriebsseitigen Teil der Kraftübertragung eine Druckfeder (14) zwischen dem Kopplungselement (4) und einem rotierbaren Abtriebselement (15) angeordnet ist, wobei das Bewegungselement zumindest bereichsweise permanentmagnetisches Material (16) enthält.
15. Bewegungsvorrichtung nach einem der vorhergehenden Aspekte, dadurch gekennzeichnet, dass ein antriebsseitiger Teil und ein abtriebsseitiger Teil der Kraftübertragungseinrichtung jeweils mindestens einen Bereich aus permanentmagnetischem Material (13, 16) enthalten, die so aufeinander bezogen sind, dass die Drehung des antriebsseitigen Teils eine Drehung des abtriebsseitigen Teils nach sich zieht.
16. Bewegungsvorrichtung nach Aspekt 15, dadurch gekennzeichnet, dass vorzugsweise antriebsseitig und/oder abtriebsseitig jeweils 1-16 Permanentmagneten vorgesehen sind.
17. Bewegungsvorrichtung nach einem der vorhergehenden Aspekte, dadurch gekennzeichnet, dass pro Kopplungselement (4) ein Motor vorgesehen ist.
18. Bewegungsvorrichtung nach Aspekt 17, dadurch gekennzeichnet, dass die einzelnen Motore unabhängig voneinander bewegbar sind.
19. Bewegungsvorrichtung nach einem der vorhergehenden Aspekte, dadurch gekennzeichnet, dass der Antrieb mindestens eine elektronische und/oder mechanische Drehmomentbegrenzung vorsieht, welche derart gestaltet ist, dass das von dem Kopplungselement (4) auf eine Mechanik (18) des medizinischen Instruments wirkende Drehmoment begrenzbar ist.
20. Bewegungsvorrichtung nach einem der vorhergehenden Aspekte, dadurch gekennzeichnet, dass eine Saug- und/oder Druckspülungsvorrichtung zur Spülung mindestens eines Lumens (19) eines medizinischen Geräts vorgesehen ist.
21. Bewegungsvorrichtung nach einem der vorhergehenden Aspekte, dadurch gekennzeichnet, dass eine Steuereinheit (20) zur Steuerung des Antriebs und/oder einer Spülvorrichtung und/oder einer Ultraschallvorrichtung vorgesehen ist.
22. Bewegungsvorrichtung nach einem der vorhergehenden Aspekte, dadurch gekennzeichnet, dass mehrere medizinische Instrumente (3) in einem Adapter (2) ankoppelbar sind.
23. Bewegungsvorrichtung nach einem der vorhergehenden Aspekte, dadurch gekennzeichnet, dass unterschiedliche Adapter (2) in die Bewegungsvorrichtung integrierbar sind und/oder untereinander austauschbar sind.
24. System, enthaltend eine Bewegungsvorrichtung nach einem der vorhergehenden Aspekte sowie ein Ultraschallbad (8), wobei das System so ausgestaltet ist, dass an die Bewegungsvorrichtung angekoppelte medizinische Instrumente (3) zumindest bereichsweise innerhalb des Ultraschallbads (8), vorzugsweise unterhalb einer Wasseroberfläche des Ultraschallbads anordenbar sind.
25. System zur Reinigung von medizinischen Instrumenten (3), enthaltend eine Bewegungsvorrichtung mit
   - einem Adapter (2) zur Ankopplung mindestens eines medizinischen Instruments (3), wobei
   - der Adapter (2) mindestens ein mittels eines Antriebs bewegbares Kopplungselement (4) umfasst, wobei das Kopplungselement (4) so gestaltet ist, dass bei Ankopplung des medizinischen Instruments (3) zumindest ein Bereich des medizinischen Instruments (3) durch Bewegung des Kopplungselements (4) in Bewegung versetzbar ist,
   - außerdem enthaltend eine Schwenk- und/oder Hebevorrichtung (21), die dergestalt ausgeführt ist, dass der Adapter (2) zwischen
      a) einem Bestückungszustand, der oberhalb des Wannenbades angeordnet ist (siehe Fig. 2A, links) und
      b) einem Reinigungszustand, bei dem der Adapter zumindest bereichsweise unter einer Wasseroberfläche angeordnet ist (siehe Fig. 2A rechts), verfahrbar ist.
26. System nach Aspekt 25, dadurch gekennzeichnet, dass unterschiedliche Adapter (2) an die Schwenk- und/oder Hebevorrichtung (21) ankoppelbar sind.
27. System nach einem der Aspekte 25 ff., dadurch gekennzeichnet, dass zwischen dem Bestückungszustand und dem Reinigungszustand eine aufgrund der Bauart der Schwenk- und/oder Hebevorrichtung eine Drehung eines angekoppelten Adapters erfolgt.
28. System nach einem der Aspekte 25 ff., dadurch gekennzeichnet, dass die Schwenk- und/oder Hebevorrichtung (21) in mindestens einer Stellung rastbar ist.
29. System nach einem der Aspekte 25 ff., dadurch gekennzeichnet, dass im Bestückungszustand medizinische Instrumente (3), von einer stehenden Bedienperson aus gesehen frontal, ankoppelbar sind.
30. System nach einem der Aspekte 24 ff., dadurch gekennzeichnet, dass im Reinigungszustand medizinische Instrumente (3) im Wesentlichen parallel zum Boden einer Wanne eines Ultraschallbads (8) anordenbar sind.
31. System nach einem der Aspekte 24 ff., dadurch gekennzeichnet, dass im Reinigungszustand medizinische Instrumente (3) derart angeordnet sind, dass zwischen Boden und/oder Wänden der Wanne sowie den medizinischen Instrumenten keine weitere Bauteile vorhanden sind.

### Bezugszeichenliste

- 1: Bewegungsvorrichtung
- 2: Adapter
- 3: medizinisches Instrument
- 4: Kopplungselement
- 5: Magnetkupplung
- 6: Antrieb
- 7: Kunststoffwand
- 8: Ultraschallbad
- 9: Kuppelteil
- 10: Instrumentenschaft
- 11: Operationsteil
- 12: rotierbares Antriebselement
- 13: permanentmagnetisches Material (antriebsseitig)
- 14: Druckfeder
- 15: rotierbares Abtriebselement
- 16: permanentmagnetisches Material (abtriebsseitig)
- 17: Motor
- 18: Mechanik des medizinischen Instruments
- 19: Lumen zur Saug- und/oder Druckspülung des medizinischen Instruments
- 20: Steuereinheit
- 21: Schwenk- und/oder Hebevorrichtung
- 22: Gasdruckfeder (= Gaszugfeder)
- 23: Rastmechanismus
- 24: Trennschicht
- 25: Kanalwähler

## Patentansprüche

1. System zur Reinigung von medizinischen Instrumenten (3), enthaltend eine Bewegungsvorrichtung mit
- einem Adapter (2) zur Ankopplung mindestens eines medizinischen Instruments (3), wobei
- der Adapter (2) mindestens ein mittels eines Antriebs bewegbares Kopplungselement (4) umfasst, wobei das Kopplungselement (4) so gestaltet ist, dass bei Ankopplung des medizinischen Instruments (3) zumindest ein Bereich des medizinischen Instruments (3) durch Bewegung des Kopplungselements (4) in Bewegung versetzbar ist,
- außerdem enthaltend eine Schwenk- und/oder Hebevorrichtung (21), die dergestalt ausgeführt ist, dass der Adapter (2) zwischen
a) einem Bestückungszustand, der oberhalb eines Wannenbades angeordnet ist, und
b) einem Reinigungszustand, bei dem der Adapter zumindest bereichsweise unter einer Wasseroberfläche angeordnet ist, verfahrbar ist.

2. System nach Anspruch 1, **dadurch gekennzeichnet, dass** die Bewegung des medizinischen Instruments (3) unter Flüssigkeitseinfluss erfolgt.

3. System nach einem der vorhergehenden Ansprüche, **dadurch gekennzeichnet dass** die Bewegungsvorrichtung mit ihrem Adapter die medizinischen Instrumente (3) so anordnet, dass diese medizinischen Instrumente zumindest bereichsweise unterhalb einer Flüssigkeitsoberfläche des Wannenbads sind.

4. System nach einem der vorhergehenden Ansprüche, **dadurch gekennzeichnet, dass** die Bewegungsvorrichtung so ausgeführt ist, dass sie in einem Wasserbad, einem Ultraschallbad (8), einem Wasserstrahl und/oder in einem Wassersprühnebel platzierbar ist.

5. System nach einem der vorhergehenden Ansprüche, **dadurch gekennzeichnet, dass** im Reinigungszustand medizinische Instrumente (3) im Wesentlichen parallel zum Boden einer Wanne eines Ultraschallbads (8) anordenbar sind und/oder die medizinischen Instrumente (3) im Reinigungszustand derart angeordnet sind, dass zwischen Boden und/oder Wänden der Wanne sowie den medizinischen Instrumenten (3) keine weiteren Bauteile vorhanden sind.

6. System nach einem der vorhergehenden Ansprüche, **dadurch gekennzeichnet, dass** eine Kraftübertragung zwischen dem Antrieb und dem Kopplungselement (4) berührungslos erfolgt.

7. System nach einem der vorhergehenden Ansprüche, **dadurch gekennzeichnet, dass** jedes Kopplungselement (4) vom Bewegungszyklus her frei verfahrbar ist und keine kinematischen Zwangsbedingungen zwischen einzelnen Kopplungselementen bestehen.

8. System nach einem der vorhergehenden Ansprüche, **dadurch gekennzeichnet, dass** durch einen einzelnen Antrieb die Kopplungselemente eines einzelnen medizinischen Instruments gemeinsam oder die Kopplungselemente für alle medizinischen Instrumente gemeinsam bewegt werden.

9. System nach einem der vorhergehenden Ansprüche, **dadurch gekennzeichnet, dass** für jedes Kopplungselement ein Maximaldrehmoment vorgesehen ist.

10. System nach einem der vorhergehenden Ansprüche, **dadurch gekennzeichnet, dass** das Kopplungselement (4) rotierbar ausgeführt ist.

11. System nach einem der vorhergehenden Ansprüche, **dadurch gekennzeichnet, dass** eine Saug- und/oder Druckspülungsvorrichtung zur Spülung mindestens eines Lumens des medizinischen Instruments (3) vorgesehen ist.

12. System nach einem der vorhergehenden Ansprüche, **dadurch gekennzeichnet, dass** das medizinische Instrument (3) ein endoskopisches Instrument mit mindestens einem beweglichen Teil ist, das nach Maßgabe eines Kopplungselements bewegbar ist.

13. System nach einem der vorhergehenden Ansprüche, **dadurch gekennzeichnet, dass** das medizinische Instrument (3) ein endoskopische Instrument ist, das mehrere unabhängig voneinander bewegbare Operationsteile aufweist, die von unterschiedlichen Kopplungselementen (4) unabhängig voneinander bewegbar sind, wobei das endoskopische Instrument vorzugsweise einen Koppelteil (9) zur Ankopplung an den Adapter aufweist, einen sich an das Koppelteil (9) anschließenden Instrumentenschaft (10) und einen an dem dem Koppelteilabgewandten Ende des Instrumentenschafts angebrachten Operationsteil (11).

14. System nach einem der vorhergehenden Ansprüche, **dadurch gekennzeichnet, dass** im Bestückungszustand medizinische Instrumente (3), von einer stehenden Bedienperson aus gesehen frontal, ankoppelbar sind.

15. System nach einem der vorhergehenden Ansprüche, **dadurch gekennzeichnet, dass** mehrere Kopplungselemente (4) in einem Adapter vorgesehen sind und/oder dadurch, dass unterschiedliche Adapter (2) an die Schwenk- und/oder Hebevorrichtung (21) ankoppelbar sind.
